Europäisches Patentamt

European Patent Office

Office européen des brevets

(19)

(11) EP 1 496 467 A2

(12) EUROPEAN PATENT APPLICATION

(43) Date of publication:
12.01.2005 Bulletin 2005/02

(51) Int Cl.⁷: $G06K\ 9/20$, $G01R\ 27/26$

(21) Application number: 04253999.9

(22) Date of filing: 02.07.2004

(84) Designated Contracting States:
AT BE BG CH CY CZ DE DK EE ES FI FR GB GR
HU IE IT LI LU MC NL PL PT RO SE SI SK TR
Designated Extension States:
AL HR LT LV MK

(30) Priority: 11.07.2003 JP 2003195951

(71) Applicant: ALPS ELECTRIC CO., LTD.
Ota-ku Tokyo 145 (JP)

(72) Inventors:
• Saito, Junichi
  Ota-ku Tokyo (JP)
• Umeda, Yuichi
  Ota-ku Tokyo (JP)

(74) Representative: Kensett, John Hinton
Saunders & Dolleymore,
9 Rickmansworth Road
Watford, Hertfordshire WD18 0JU (GB)

(54) Capacitive sensor

(57)    A pressure-sensitive capacitive sensor (1) includes a sensing unit (2) in which a plurality of column wires ($S_j$) and a plurality of row wires ($V_i$) are formed in a matrix, a detecting signal generator (3), and filters ($4_i$). Capacitances ($C_{ij}$) at intersections between the column wires and the row wires change in accordance with externally applied pressure. The detecting signal generator sequentially outputs pulse signals of a predetermined frequency to the column wires of the sensing unit. The filters are connected to the respective row wires of the sensing unit and extract amplitudes of signals of the predetermined frequency. The amplitude is proportional to the capacitance at the intersection.

FIG. 1

EP 1 496 467 A2

**Description**

BACKGROUND OF THE INVENTION

1. Field of the Invention

The present invention relates to a capacitive sensor mainly used as a fingerprint sensor.

2. Description of the Related Art

A pressure-sensitive capacitive sensor has been known as a fingerprint sensor, which is most promising in biometric security applications, such as a biometric identification. Such a pressure-sensitive capacitive sensor has two films respectively having column wires and row wires at predetermined pitches on their surfaces, and an insulating layer between the films having a predetermined distance. In the pressure-sensitive capacitive sensor, when a finger touches the film, the film is deformed in accordance with the shape of the fingerprint and the spacing between the column wires and row wires varies depending on the position on the film. Thus, the shape of the fingerprint is detected from capacitances at intersections of the column wires and row wires. In a known technology, to detect a small capacitance at the less than several-hundred femtofarad (fF) level, a detecting circuit is used in which the capacitance is converted to an electrical signal by a switched capacitor circuit. In the detecting circuit, a capacitive sensor element that is driven by a first driving signal and detects the capacitance of a target object, and a reference capacitive element that is driven by a second driving signal to generate a reference capacitance for the detecting circuit are connected to a common switched capacitor circuit. First and second sample-and-hold units are alternatively operated to sample output signals from the elements, respectively. The detecting circuit calculates a difference between the sampling results and then outputs it as a detecting signal.

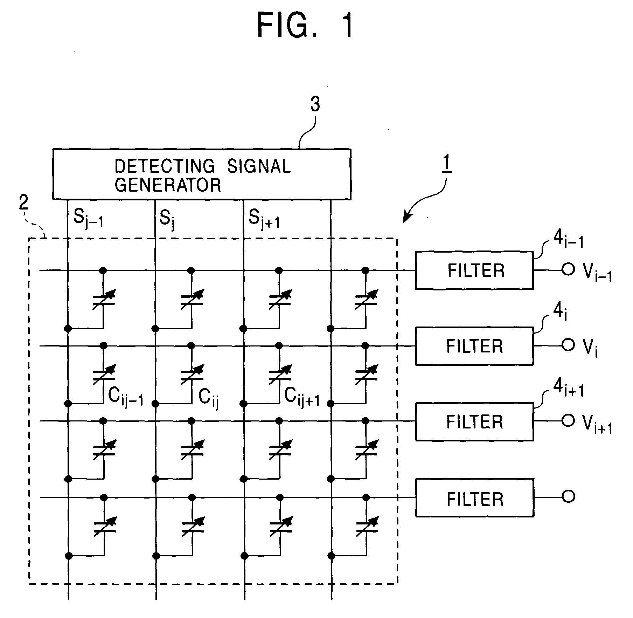

In the common switched capacitor circuit of the detecting circuit, since a capacitance Cs to be detected is inversely proportional to a feedback capacitance Cf, a reliable detection is achieved. In addition, this structure cancels the effect (feed-through) of leakage of an electric charge Qd retained in the parasitic capacitors between a gate electrode and other electrodes of a reset switch (feedback control switch) of the switched capacitor circuit to the other electrodes. Furthermore, some of an offset component of a reference voltage of the switched capacitor circuit and low-frequency noise of input signals can be eliminated by calculating the difference between two sampling results (refer to, for example, Japanese Unexamined Patent Application Publication No. 8-145717 corresponding to U.S. Pat. No. 5633594, in particular, paragraphs 0018 to 0052 and Figs. 1 to 4).

Unfortunately, in the above-described detecting circuit of the pressure-sensitive capacitive sensor, when a small sensor capacitance Cs is measured, since an output voltage of the switched capacitor circuit is inversely proportional to the feedback capacitance Cf, the capacitance Cf must be small to obtain a large output voltage. Therefore, an operational amplifier is used in a mode almost the same as the open loop mode. Accordingly, a significant amount of noise from the wires, the human body, and a power supply appears. Additionally, even if the circuit is completely shielded, a required electrical current for maintaining a negative input at a predetermined voltage level makes the output voltage of the amplifier unstable. Furthermore, when the reset switch is open, a leakage current decreases the electric charge of the capacitance Cf. If the charge Cf becomes small, the decrease in the charge cannot be neglected. Also, a feed-through effect of the reset switch becomes large and, therefore, a voltage higher than the power supply voltage of the operational amplifier is output and the output voltage is saturated to make the detection difficult.

Thus, the measurement of the capacitance is disadvantageously difficult.

SUMMARY OF THE INVENTION

Accordingly, it is an object of the present invention to provide a capacitive sensor capable of reliably detecting a small capacitance by preventing the effect of noise, and by preventing a leakage current and feed-through of a switching transistor.

According to an aspect of the present invention, a pressure-sensitive capacitive sensor includes a sensing unit, a signal output unit, and a plurality of filters. The sensing unit includes a plurality of column wires and a plurality of row wires in a matrix, capacitances at intersections between the column wires and the row wires change in accordance with externally applied pressure, and the sensing unit detects changes in the capacitances at the intersections and a distribution of the externally applied pressure based on the detecting result of the changes. The signal output unit sequentially outputs pulse signals of a predetermined frequency to the column wires of the sensing unit. The plurality of filters are connected to the respective row wires of the sensing unit and extract signals of the predetermined frequency from signals received from the respective row wires.

According to the configuration, only signals of a predetermined frequency are extracted by the filter and am-

**EP 1 496 467 A2**

plitudes of the signals are detected. Accordingly, various types of noise can be reduced. Additionally, since the configuration does not require a reset switch, charge loss in a feedback capacitor due to a leakage current is prevented and the effect of feed-through, whereby the electric charge in a gate electrode leaks, is also prevented. As a result, the sensor can reliably detect a small change in the capacitance.

According to a further aspect of the present invention, a pressure-sensitive capacitive sensor includes a sensing unit, a signal output unit, a selector, and a filter. The sensing unit includes a plurality of column wires and a plurality of row wires in a matrix, capacitances at intersections between the column wires and the row wires change in accordance with externally applied pressure, and the sensing unit detects changes in the capacitances at the intersections and a distribution of the pressure based on the detecting result of the changes. The signal output unit sequentially outputs pulse signals of a predetermined frequency to the column wires of the sensing unit. The selector sequentially selects and outputs signals received from the respective row wires of the sensing unit, and the filter extracts signals of the predetermined frequency from the signals output from the selector.

According to the configuration, a single filter is selectively connected to row wires instead of a plurality of filters connected to respective row wires. As a result, problems caused by variations of filters can be eliminated and the sizes of subsequent circuit blocks can be reduced.

According to another aspect of the present invention, a capacitive sensor includes a sensing unit, a signal output unit, and a plurality of filters. The sensing unit includes a plurality of column wires and a plurality of row wires in a matrix, capacitances in the vicinity of intersections between the column wires and the row wires change in accordance with irregularities on a surface of a measuring object distant from the sensing unit by a short distance, and the sensing unit detects changes in the capacitances in the vicinity of the intersections and the irregularities of the measuring object based on the detecting result of the changes. The signal output unit sequentially outputs pulse signals of a predetermined frequency to the column wires of the sensing unit. The plurality of filters are connected to the respective row wires of the sensing unit and extracts signals of the predetermined frequency from signals received from the respective row wires.

According to the configuration, since electrostatic induction changes capacitances in the vicinity of the intersections between the column wires and the row wires simply by a measuring object, which has irregularities on its surface, getting close to the sensing unit without touching, the sensor receives little stress and, therefore, the lifetime of the sensor can be prolonged.

According to another aspect of the present invention, a capacitive sensor includes a sensing unit, a signal output unit, a selector, and a filter. The sensing unit includes a plurality of column wires and a plurality of row wires in a matrix, capacitances in the vicinity of intersections between the column wires and the row wires change in accordance with irregularities on a surface of a measuring object distant from the sensing unit by a short distance, and the sensing unit detects changes in the capacitances in the vicinity of the intersections and the irregularities of the measuring object based on the detecting result of the changes. The signal output unit sequentially outputs pulse signals of a predetermined frequency to the column wires of the sensing unit. The selector sequentially selects and outputs signals received from the respective row wires of the sensing unit, and the filter extracts signals of the predetermined frequency from the signals output from the selector.

According to the configuration, since electrostatic induction changes capacitances in the vicinity of the intersections between the column wires and the row wires simply by a measuring object, which has irregularities on its surface, getting close to the sensing unit without touching, the sensor receives little stress and, therefore, the lifetime of the sensor can be prolonged.

Preferably, the filter includes a first capacitor disposed between an input terminal and the ground, an amplifier, a first resistor disposed between the input terminal and an output terminal of the amplifier, a second resistor disposed between the input terminal and an inverting input terminal of the amplifier, and a second capacitor disposed between the inverting input terminal and an output terminal of the amplifier.

In this configuration, a bias voltage fed back in terms of a direct current is applied to the inverting input terminal of the amplifier, thus providing a stable operation.

Preferably, a capacitor is connected to an input terminal of the filter in series.

In this configuration, low-frequency noise occurring between the sensing unit and the filter can be reduced.

Embodiments of the invention will now be described, by way of example only, with reference to the accompanying drawings, in which:

Fig. 1 is a block diagram of a first embodiment of the present invention;
Fig. 2 is a circuit diagram of a filter according to the embodiment;
Fig. 3 is a timing diagram of the operation of the embodiment;
Fig. 4 is a graph illustrating the effect of a capacitance Cy on the filter, where Cy is a total capacitance of unselected wires;
Fig. 5 is a block diagram of a relevant portion of a second embodiment of the present invention;

3

Fig. 6 is a block diagram of the entire configuration of the embodiment;
Fig. 7 is a timing diagram of the operation of the embodiment;
Fig. 8 is a diagram of an equivalent circuit of the circuit shown in Fig. 5;
Fig. 9 is a diagram of another equivalent circuit of the circuit shown in Fig. 8;
Fig. 10 is a configuration in which a capacitor C3 is connected to the input terminal of a filter shown in Fig. 1 or Fig. 5;
Fig. 11 is a top view of a sensing unit according to a third embodiment of the present invention;
Fig. 12 is a cross-sectional view of the sensing unit; and
Fig. 13 is a diagram for explaining the operation of the sensing unit.

DESCRIPTION OF THE PREFERRED EMBODIMENTS

A first embodiment of the present invention will now be described with reference to the accompanying drawings.

Fig. 1 is a block diagram of a capacitive sensor 1 according to the embodiment. The capacitive sensor 1 includes a sensing unit 2 with which a target object, for example, a fingertip is brought into contact; a detecting signal generator 3 which outputs detecting signals to the sensing unit 2; filters 4i-1, 4i, 4i+1, ... which receive output signals from the sensing unit 2; and a processing circuit (not shown) which processes outputs from the filters 4i-1, 4i, 4i+1, ....

The sensing unit 2 has first and second opposing flexible thin plates with a small spacing therebetween. A plurality of column wires are evenly formed on the first thin plate, while a plurality of row wires are evenly formed on the second thin plate in the direction perpendicular to the column wires. Urging a fingertip onto the sensing unit 2 changes the spacings between the column wires and the row wires at their intersections, thus changing the capacitances at the intersections in accordance with the irregularity of the fingerprint.

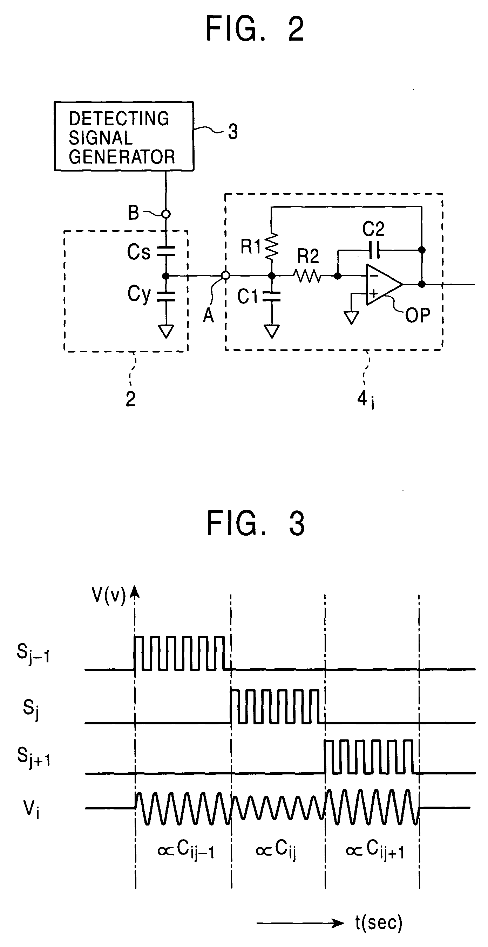

The detecting signal generator 3 sequentially outputs pulse signals to the column wires Sj-1, Sj, Sj+1, ... in the sensing unit 2, as shown in Fig. 3. In this case, the pulse signals output to the column wires Sj-1, Sj, Sj+1, ... are identical. During outputting the pulse signal to one of the column wires, the detecting signal generator 3 outputs the ground potential to the other column wires.

The filters 4i-1, 4i, 4i+1, ... have the same structure. Each filter is a circuit that extracts a signal of a predetermined frequency from a signal delivered to the corresponding row wire in the sensing unit 2, that is, that extracts a signal output from the detecting signal generator 3 and transmitted from a column wire to the corresponding row wire. Fig. 2 is a detailed circuit configuration of the filter 4i. An input terminal A of the filter 4i is connected to the row wire. Also, the input terminal A is connected to an inverting input terminal of an operational amplifier OP via a resistor R2 and is grounded via a capacitor C1. A noninverting input terminal of the operational amplifier OP is grounded. An output terminal of the operational amplifier OP is connected to the inverting input terminal via a capacitor C2 and is also connected to the input terminal A via a resistor R1.

The operation of the above-described capacitive sensor 1 will now be described with reference to a wave form chart in Fig. 3.

The detecting signal generator 3 outputs a pulse signal to the column wire Sj-1 and outputs the ground potential to the other column wires Sj and Sj+1. The pulse signal output to the column wire Sj-1 is delivered to every row wire through a capacitor at an intersection between the column wire and the row wire. That is, as shown in Fig. 3, as the capacitance at the intersection increases, the amplitude of a signal delivered to the row wire increases. As a capacitance at the intersection decreases, the amplitude of the signal delivered to the row wire decreases. The signals delivered to the row wires are extracted by the filters 4i-1, 4i, 4i+1, ..., and are then output to a processing circuit. The processing circuit converts peak values of the signals extracted by the filters 4i-1, 4i, 4i+1 ... to digital data and stores them in a memory. Thus, data corresponding to the capacitances at the intersections between the column wire Sj-1 and the row wires are stored in the memory.

Subsequently, the detecting signal generator 3 outputs a pulse signal to the column wire Sj. The filters 4i-1, 4i, 4i+1, ... deliver the signals from the respective row wires to the processing circuit. Thus, data corresponding to the capacitances at the intersections between the column wire Sj and the row wires are stored in the memory. The above-described process is repeated so that all the capacitances at the intersections between the column wires and the row wires are stored in the memory. Accordingly, irregularities on the surface of the sensing unit 2 can be visualized by displaying the data in the memory. As a result, by recording data in the above-described manner with a user's fingertip urged onto the sensing unit 2, data on the fingerprint of the user's fingertip can be stored and displayed.

A filter viewed from the input terminal A in Fig. 2 exhibits the configuration of a low pass filter. However, the configuration viewed from a driving terminal B of the detecting signal generator 3 can be approximated by the following band pass filter. A transfer function $A(j\omega)$ of the filter is given by:

(Formula 1)

$$A(j\omega) = \cfrac{-\cfrac{Cs}{C2} \bullet \cfrac{1}{1 + \cfrac{R2}{R1}} \bullet \cfrac{1}{Q}\left(j\cfrac{\omega}{\omega0}\right)}{\left(j\cfrac{\omega}{\omega0}\right)^2 + \cfrac{1}{Q} \bullet \left(j\cfrac{\omega}{\omega0}\right) + 1 - \left(\cfrac{\omega}{\omega0}\right) \bullet \left(\cfrac{Cs}{C1}\right)} \qquad \cdots (1)$$

where

(Formula 2)

$$\omega0 = \cfrac{1}{\sqrt{C1 \bullet C2 \bullet R1 \bullet R2}} \qquad\qquad (2)$$

$$\cfrac{1}{Q} = \left(\cfrac{1}{R1} + \cfrac{1}{R2}\right) \bullet \cfrac{\sqrt{C1 \bullet C2 \bullet R1 \bullet R2}}{C1} \qquad \cdots (3)$$

$$L = -\cfrac{Cs}{C2} \bullet \cfrac{1}{1 + \cfrac{R2}{R1}} \qquad\qquad (4)$$

When

(Formula 3)

$$s = j\cfrac{\omega}{\omega_0} \qquad\qquad (5)$$

A(jω) is given by:

(Formula 4)

$$A(j\omega) = \cfrac{-L \bullet \cfrac{1}{Q} \bullet s}{s^2 + \cfrac{1}{Q} \bullet s + 1 - \left(\cfrac{\omega}{\omega0}\right) \bullet \left(\cfrac{Cs}{C1}\right)} \qquad \cdots (6)$$

In this case, since this circuit is used at the center frequency of the filter,

(Formula 5)

$$\cfrac{\omega}{\omega_0} \approx 1 \qquad\qquad (7)$$

In addition, since Cs is 150 fF and C1 is several-hundred pF,

(Formula 6)

$$\frac{Cs}{C1} = 10^{-3} \tag{8}$$

Therefore,

**(Formula 7)**

$$\left(\frac{\omega}{\omega 0}\right) \frac{Cs}{C1} \lll 1 \quad \cdots (9)$$

Thus, A(jω) is represented by the following approximation:

(Formula 8)

$$A(j\omega) = \frac{-L \cdot \frac{1}{Q} \cdot s}{S^2 + \frac{1}{Q} \cdot s + 1} \tag{10}$$

This equation represents a transfer function of a band pass filter. By this approximation, the amplitude characteristic A(jω) can be regarded as a transfer characteristic of a band pass filter (BPF).

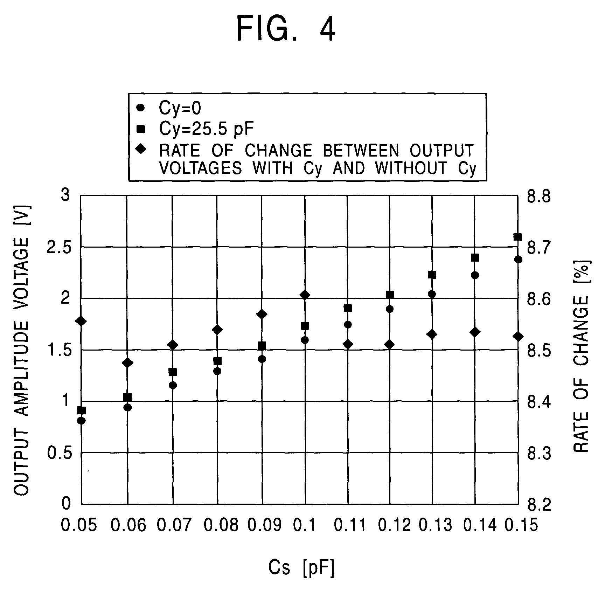

In this case, as shown in Fig. 2, a total capacitance Cy, which is a total capacitance of capacitors connected to the column wires having the ground potential (for example, 100 fF × 255 = 25.5 pF), is added to the capacitor C1 (for example, 150 pF) in parallel. However, as described above, since the variation in cutoff frequency caused by the variation of the capacitance of the capacitor C1 is reduced, the effect of Cy on the filter characteristic is eliminated. Fig. 4 shows the experimental result. As can be seen from the result, the entire curve of the output voltage is shifted with the linearity being maintained regardless of the amount of capacitance Cy, that is, regardless of external pressure. Additionally, a scanning time in the column direction (about 0.1 second) is shorter than a time for the fingertip to remain unmoved (about 0.5 second). Consequently, Cy stays constant during a scan, thus eliminating any effect on the measurement value.

Thus, according to the embodiment, since only a predetermined frequency is extracted from the output signal by the filter and the amplitude is detected, various types of noise are reduced. Also, the capacitances are measured without the feed-through effect in the reset switch.

A second embodiment of the present invention will now be described.

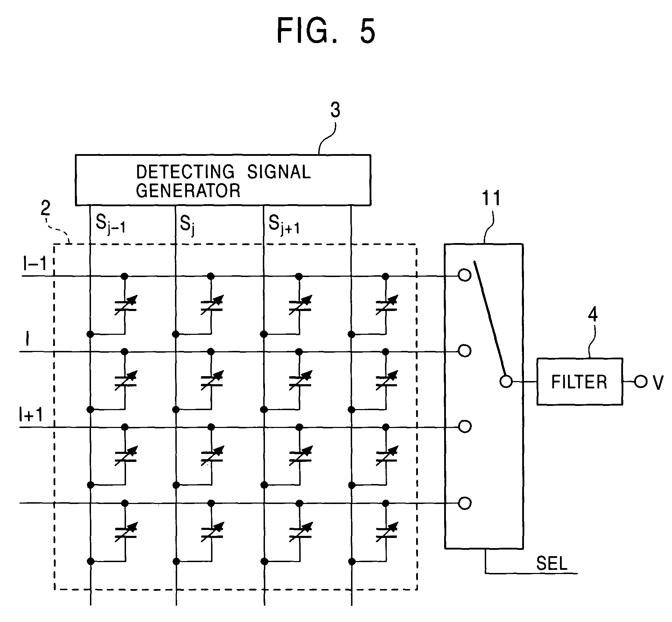

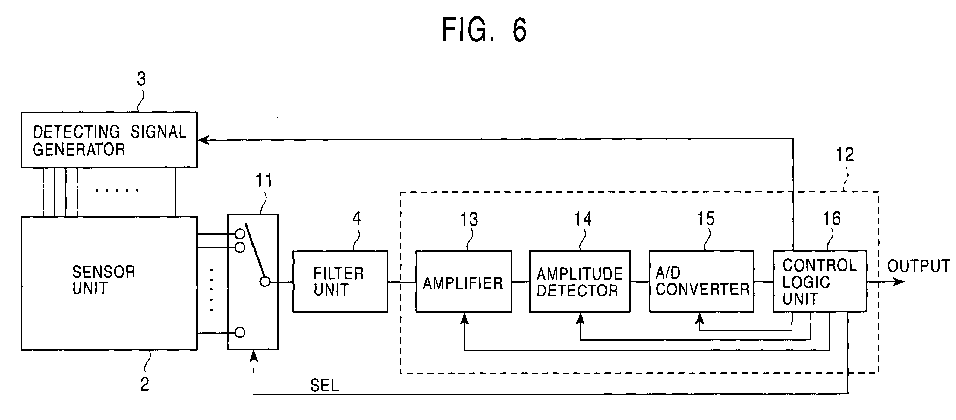

Figs. 5 and 6 are block diagrams of a capacitive sensor according to the second embodiment. Elements identical to those illustrated and described in relation to Fig. 1 are designated by like reference numerals. In Fig. 5, a sensing unit 2 and a detecting signal generator 3 have the same configurations as those having like reference numerals in Fig. 1. A filter 4 has the same configuration as each of the filters 4i-1, 4i, 4i+1, ... shown in Fig. 1. A selector 11 selects one of the row wires based on a select signal SEL and connects the wire to an input terminal of the filter 4.

Fig. 6 is a configuration of a capacitive sensor having a control circuit 12 in addition to the above-described configuration. In the control circuit 12, an amplifier 13 amplifies an output signal from the filter 4 and outputs it. An amplitude detector 14 sequentially outputs analog signals corresponding to amplitudes of signal waves sequentially output from the amplifier 13. An A/D converter 15 converts the analog signals sequentially output from the amplitude detector 14 to digital data and output them to a control logic unit 16. The control logic unit 16 stores the digital data in an internal memory and outputs the stored data to a display unit (not shown). Additionally, the control logic unit 16 outputs control signals to control the detecting signal generator 3, the selector 11, the amplifier 13, the amplitude detector 14, and the A/D converter 15.

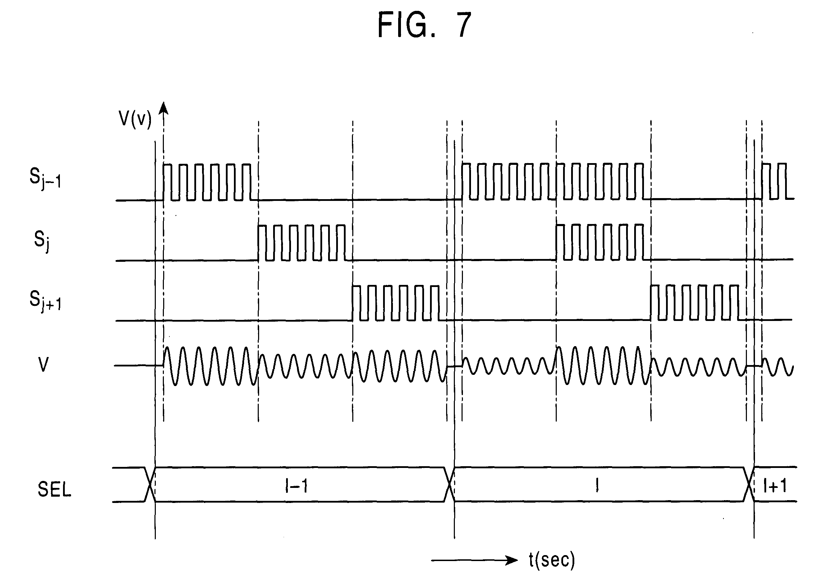

The operation of the above-described embodiment will now be described with reference to a wave form chart shown in Fig. 7.

For a capacitance measurement, the control logic unit 16 first outputs the select signal SEL to the selector 11 to select a row wire I-1 in the sensing unit 2. The selector 11 receives the select signal to connect the row wire I-1 to the input terminal of the filter 4. Then, the control logic unit 16 outputs a start signal to the detecting signal generator 3. Upon reception of the start signal, the detecting signal generator 3 first outputs a pulse signal to the column wire Sj-1, and then, after a predetermined amount of time, outputs a pulse signal to the column wire Sj. Likewise, at predetermined intervals, the detecting signal generator 3 sequentially outputs a pulse signal to the column wire Sj+1, .... As in the first embodiment, the detecting signal generator 3 outputs the ground potential to other column wires that do not receive the pulse signal.

Thus, as shown in Fig. 7, a pulse signal that is output from the detecting signal generator 3 and passes through a capacitor at an intersection between the column wire Sj-1 and the row wire I-1 is first output from the filter 4. A pulse signal which passes through a capacitor at an intersection between the column wire Sj and the row wire I-1 is then output. Likewise, pulse signals that pass through capacitors at intersections between the subsequent column wires and the row wire I-1 are sequentially output from the filter 4. The pulse signal output from the filter 4 is amplified by the amplifier 13. The amplitude of the pulse signal is then detected by the amplitude detector 14 and the detected value is converted to digital data by the A/D converter. The digital data is then input to the control logic unit 16. The control logic unit 16 stores the sequentially input data in the memory. Thus, data corresponding to the capacitances at the intersections along the row wire I-1 are stored in the memory.

Subsequently, upon completion of storing all data at the intersections along the row wire I-1 in the memory, the control logic unit 16 outputs the select signal SEL to the selector 11 in order to select the row wire I. Upon reception of the select signal, the selector 11 connects the row wire I to the input terminal of the filter 4. On the other hand, after the detecting signal generator 3 outputs the pulse signals to all the column wires for the row wire I-1, the detecting signal generator 3 returns to the column wire Sj-1 and sequentially outputs pulse signals to the column wires Sj-1, Sj, Sj+1, .... Accordingly, pulse signals passing through the intersections along the row wire I are sequentially output from the filter 4. Digital data representing amplitudes of the signals are stored in the memory of the control logic unit 16. The same process is repeated until data corresponding to capacitances at all intersections in the sensing unit 2 are stored in the memory of the control logic unit 16.

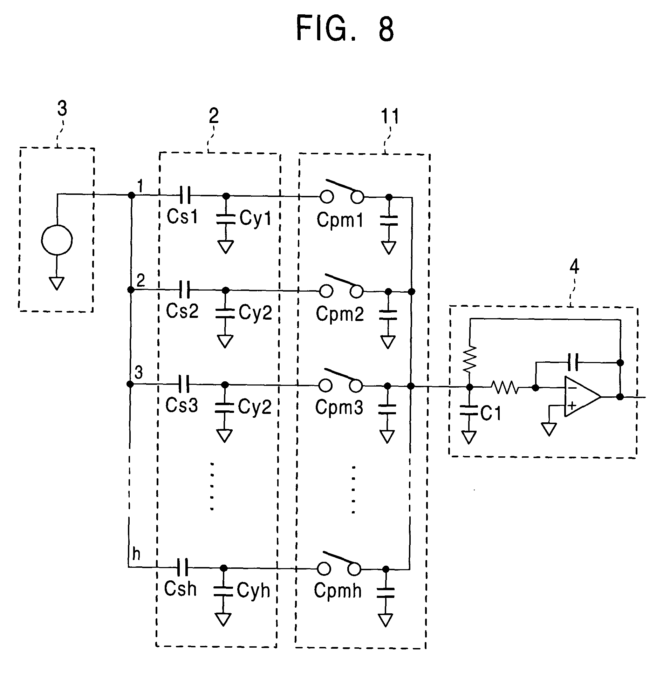

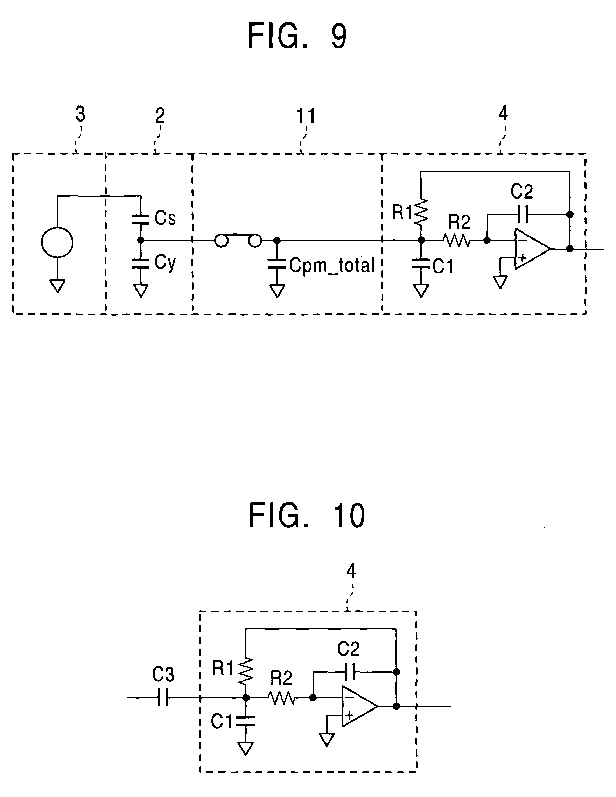

Fig. 8 is a diagram of an equivalent circuit of the circuit shown in Fig. 5. The selector 11 (multiplexer) has an output parasitic capacitance of about Cpm per channel. Accordingly, an h-stage selector has a parasitic capacitance of h times Cpm. Fig. 9 is a diagram of an equivalent circuit of the circuit when the total capacitance is Cpm_total. This capacitance can be included in the capacitor C1 of the filter 4.

Thus, according to the embodiment, a single filter is selectively connected to row wires. As a result, problems caused by variations of filters can be eliminated and the size of a circuit block can be reduced.

Additionally, in the first and second embodiments, a capacitor C3 may be connected to the input terminal of the filter 4 or to the filters 4i-1, 4i, 4i+1, ..., as shown in Fig. 10. This configuration significantly decreases low-frequency noise occurring between the sensing unit 2 and the filter. In this case, for example, the capacitance value of the capacitor preferably ranges from 10 to 100 pF mainly for cutting the noise at 50 to 60 Hz. From a qualitative point of view, since the capacitor C3 has a large capacitance value, the capacitor C3 functions as almost a short circuit in an alternating current environment. From a quantitative point of view, a total capacitance Csym of Cs and Cy will be discussed. The capacitance Csym is given by:

(Formula 9)

$$Csym = \frac{Cs \cdot C3}{Cs + C3} = \frac{Cs}{1 + \dfrac{Cs}{C3}} \qquad (11)$$

In this equation, since Cs is 150 fF and C3 is 100 pF,

(Formula 10)

$$\frac{Cs}{C3} = 10^{-3} \qquad (12)$$

Therefore, $Csym \cong Cs$. Consequently, Cs is unaffected by C3.

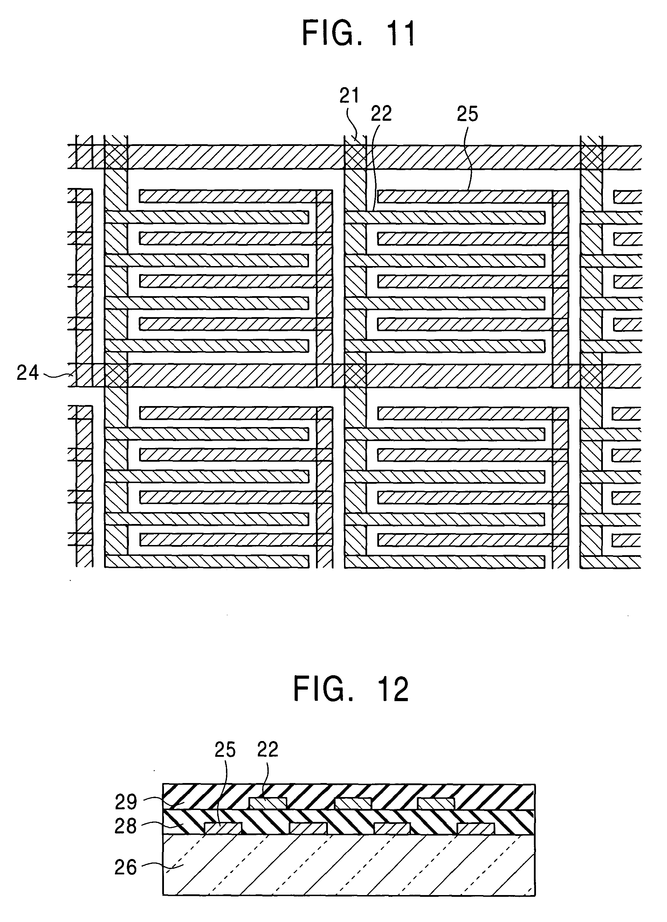

A third embodiment of the present invention will now be described. Fig. 11 is a top view of electrodes. Second comb-shaped electrodes 22 extend from a column wire 21, while first comb-shaped electrodes 25 extend from a row wire 24. Fig. 12 is a cross-sectional view of the electrodes. The second electrodes 22 are formed on a different plane from the first electrodes 25. The first electrodes 25 are formed on a glass substrate 26 and are covered with a first insulating film 28. The second electrodes 22 are formed on the first insulating film 28 and are covered with a second insulating film 29. If these wires and electrodes are made of, for example, indium tin oxide (ITO), which is transparent, and the first insulating film 28 and the second insulating film 29 are made of silicon nitride (SiNx), the detecting device can be light-transmitting.

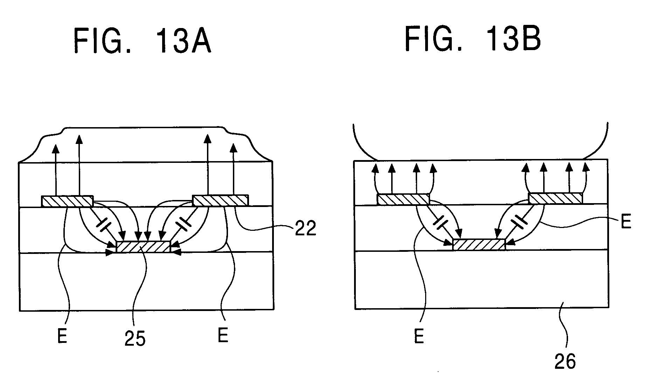

Figs. 13A and 13B illustrate a mechanism by which the electric capacitance between the second electrodes 22 and the first electrodes 25 changes. Fig. 13A shows the distribution of electric flux lines E at a fingerprint valley. As shown in Fig. 13B, when a fingerprint ridge of a human fingertip, which is a dielectric material, moves towards the second electrode 22, some of the electric flux lines emanating from the second electrodes 22 are attracted by the fingertip due to electrostatic induction instead of going to the first electrode 25. Accordingly, the capacitance between the second electrode 22 and the first electrode 25 is decreased. Thus, according to the third embodiment, the capacitance between the electrodes changes by lightly pressing the fingertip onto the sensing unit instead of firmly pressing the fingertip onto the sensing unit. Therefore, the fingerprint can be recognized by detecting the capacitance change using the above-described method.

As described above, according to this embodiment, the sensor is not stressed since electrostatic induction changes the capacitance by simply pressing a dielectric measuring object having irregularities on its surface onto the sensing unit.

If the second electrodes 22 overlap the first electrode 25, although the human fingertip produces electrostatic induction, the electric flux lines E are trapped between the overlapping areas of the two electrodes. This reduces the change in electric capacitance. Accordingly, the two electrodes must not be overlapped.

**Claims**

**1.** A pressure-sensitive capacitive sensor comprising:

a sensing unit comprising a plurality of column wires and a plurality of row wires in a matrix, capacitances at intersections between the column wires and the row wires changing in accordance with externally applied pressure, the sensing unit detecting changes in the capacitances at the intersections and a distribution of the externally applied pressure based on the detecting result of the changes;
a signal output unit for sequentially outputting pulse signals of a predetermined frequency to the column wires of the sensing unit; and
a plurality of filters connected to the respective row wires of the sensing unit and extracting signals of the predetermined frequency from signals received from the respective row wires.

**2.** A pressure-sensitive capacitive sensor comprising:

a sensing unit comprising a plurality of column wires and a plurality of row wires in a matrix, capacitances at intersections between the column wires and the row wires changing in accordance with externally applied pressure, the sensing unit detecting changes in the capacitances at the intersections and a distribution of the pressure based on the detecting result of the changes;
a signal output unit for sequentially outputting pulse signals of a predetermined frequency to the column wires of the sensing unit;
a selector for sequentially selecting and outputting signals received from the respective row wires of the sensing unit; and
a filter for extracting signals of the predetermined frequency from the signals output from the selector.

**3.** A capacitive sensor, comprising:

a sensing unit comprising a plurality of column wires and a plurality of row wires in a matrix, capacitances in the vicinity of intersections between the column wires and the row wires changing in accordance with irregularities on a surface of a measuring object distant from the sensing unit by a short distance, the sensing unit detecting changes in the capacitances in the vicinity of the intersections and the irregularities of the measuring object based on the detecting result of the changes;
a signal output unit for sequentially outputting pulse signals of a predetermined frequency to the column wires of the sensing unit; and
a plurality of filters connected to the respective row wires of the sensing unit and extracting signals of the predetermined frequency from signals received from the respective row wires.

**4.** A capacitive sensor, comprising:

a sensing unit comprising a plurality of column wires and a plurality of row wires in a matrix, capacitances in the vicinity of intersections between the column wires and the row wires changing in accordance with irregularities on a surface of a measuring object distant from the sensing unit by a short distance, the sensing unit detecting changes in the capacitances in the vicinity of the intersections and the irregularities of the measuring object based on the detecting result of the changes;

a signal output unit for sequentially outputting pulse signals of a predetermined frequency to the column wires of the sensing unit;

a selector for sequentially selecting and outputting signals received from the respective row wires of the sensing unit; and

a filter for extracting signals of the predetermined frequency from the signals output from the selector.

5.  The capacitive sensor according to one of Claims 1 to 4, wherein the filter comprises a first capacitor disposed between an input terminal and the ground, an amplifier, a first resistor disposed between the input terminal and an output terminal of the amplifier, a second resistor disposed between the input terminal and an inverting input terminal of the amplifier, and a second capacitor disposed between the inverting input terminal and an output terminal of the amplifier.

6.  The capacitive sensor according to one of Claims 1 to 4, wherein a capacitor is connected in series to an input terminal of the filter.

EP 1 496 467 A2

# FIG. 1

# FIG. 2

DETECTING
SIGNAL
GENERATOR ～3

B ～

Cs

Cy

A

R1

R2

C1

C2

OP

2

$4_i$

# FIG. 3

V(v)

$S_{j-1}$

$S_j$

$S_{j+1}$

$V_i$

$\propto C_{ij-1}$   $\propto C_{ij}$   $\propto C_{ij+1}$

⟶ t(sec)

# FIG. 4

# FIG. 5

# FIG. 6

SENSOR UNIT 2

DETECTING SIGNAL GENERATOR 3

11

FILTER UNIT 4

AMPLIFIER 13

AMPLITUDE DETECTOR 14

A/D CONVERTER 15

CONTROL LOGIC UNIT 16

12

OUTPUT

SEL

EP 1 496 467 A2

# FIG. 7

EP 1 496 467 A2

# FIG. 8

# FIG. 9

# FIG. 10

# FIG. 11

# FIG. 12

## FIG. 13A

## FIG. 13B